Europäisches Patentamt

European Patent Office

Office européen des brevets

(19)

(11) Veröffentlichungsnummer: **0 189 540**
**B1**

(12) **EUROPÄISCHE PATENTSCHRIFT**

(45) Veröffentlichungstag der Patentschrift: **13.06.90**

(51) Int. Cl.⁵: **A 61 K 6/08, A 61 K 6/02**

(21) Anmeldenummer: **85114812.2**

(22) Anmeldetag: **21.11.85**

(54) **Röntgenopaker dentalwerkstoff.**

(30) Priorität: **26.01.85 DE 3502594**

(43) Veröffentlichungstag der Anmeldung:
**06.08.86 Patentblatt 86/32**

(45) Bekanntmachung des Hinweises auf die
Patenterteilung:
**13.06.90 Patentblatt 90/24**

(84) Benannte Vertragsstaaten:
**AT CH DE FR GB IT LI SE**

(56) Entgegenhaltungen:
**EP-A-0 011 735**
**DE-A-2 458 380**
**DE-A-2 935 810**

(73) Patentinhaber: **Etablissement Dentaire IVOCLAR**
**FL-9494 Schaan (LI)**

(72) Erfinder: **Michl, Rudolf J., Dr.**
**Im Fetzer 49**
**FL-9494 Schaan (LI)**
Erfinder: **Rheinberger, Volker M., Dr.**
**Schloss-Strasse 130**
**FL-9494 Vaduz (LI)**
Erfinder: **Ott, Gilbert**
**Feldkirchstrasse 63**
**FL-9494 Schaan (LI)**

(74) Vertreter: **UEXKÜLL & STOLBERG**
**Patentanwälte**
**Beselerstrasse 4**
**D-2000 Hamburg 52 (DE)**

# EP 0 189 540 B1

**Beschreibung**

Die Erfindung betrifft röntgenopake Dentalwerkstoffe wie z.B. Füllungsmaterialien, Dentalzemente, Kronen- und Brückmaterialien, Prothesenmaterialien sowie deren Verwendung zur Herstellung von künstlichen Zähnen, Inlays, Implantaten und Fertigteilen.

Aus der DE—OS 24 58 380 sind Zahnfüllmassen bekannt, die als röntgenopaken Füllstoff Oxide und/oder Carbonate von Lanthan, Hafnium, Strontium und/oder Tantel enthalten. Aus dieser Veröffentlichung geht ferner hervor, daß es bekannt ist, daß Elemente mit hohem Atomgewicht Röntgenstrahlen stärker absorbieren. Ihrer Anwendbarkeit in der Umgebung des menschlichen Gewebes sind jedoch Grenzen gesetzt, da vielle dieser schweren Elemente toxisch oder radioaktiv sind.

Aus der DE—OS 24 20 351 sind ebenfalls Zahnfüllmaterialien bekannt, die als röntgenstrahlenundurchlässiges Material Bariumsulfat, Tantal, Jodalphionsäure, Iopanionsäure oder Ipodoinsäure enthalten können. Ferner wird auf die übrigen in Kirk-Othermer, Encyclopedia of Chemical Technology, 2. Auflage, Band 17, S. 130—141 beschriebenen röntgenstrahlenundurchlässigen Materialien verwiesen.

In der EP—PS 11 735 werden Zahnfüllmassen beschrieben, die als Röntgenkontrastmittel Verbindungen von Barium, Wismut, Lanthan, Thorium sowie Seltenen Erdmetallen (SE) enthalten. Vorzugsweise wird Bariumsulfat verwendet.

Aus anderen Veröffentlichungen ist bekannt, Methacrylatteilchen aliphatische Halogenverbindungen wie z.B. Tetrabromethan, Bromofrom, Ethyljodid, Jodbenzol etc. einzuverleiben. Dies geschieht durch Suspensionspolymerisation eines Methacrylsäurealkylesters. Dabei kann zusätzlich zur Verstärkung der Röntgenopatität an den Perlen ein anhaftendes Pigment einer Schwermetallverbindung vorhanden sein.

Ferner werden in der Literatur Dentalwerkstoffe erwähnt, die z.B. Zinnverbindungen, Bariumgläser, Bariumsulfat etc. als röntgenopakes Mittel enthalten. Weiterin werden in der US—A—3 971 754 Glaskeramiken als Füllstoffe für Zahnfüllungsmassen beschrieben, die Oxide, Carbonate oder Fluoride bestimmter Metalle enthalten, darunter Lanthanoxid und Lanthancarbonat. In der DE—OS 29 35 810 heißt es, daß zwar die Oxide der Seltenen Erden (Elemente 57 bis 71) als für Röntgenstrahlen undurchlässig vorgeschlagen wurden, daß dabei aber Probleme wegen unerwünschter Verfärbung auftauchen.

Es wurde auch schon vorgeschlagen, als Füllstoff für einen Dentalwerkstoff Calcium-, Strontium-, Barium-, Lanthan-, SE-, Tantal- und/oder Hafniumaluminosilikat mit Zeolithstruktur zu verwenden.

Es sind ferner röntgenopake Füllungsmaterialien bekannt, deren Füllstoff aus einer Mischung von mikrofeinen Siliciumdioxid und röntgenopaken Gläsern besteht, wobei die Gläser eine mittlere Korngröße über 1 µm aufweisen. Allen bis jetzt bekannten röntgenopaken mikrogefüllten Dentalwerkstoffen haftet der Mangel an, daß die Röntgenopazität noch unbefriedigend ist. Ferner ist die Transparenz der bisherigen röntgenopaken mikrogefüllten Dentalwerkstoffen unbefriedigend und die Hochglanzplierbarkeit nicht ausreichend.

Aufgabe der Erfindung ist deshalb die Schaffung eines mikrogefüllten Dentalwerkstoffes, der eine gute Röntgenopazität bei gleichzeitiger guter Transparenz aufweist, wobei die physikalischen Eigenschaften nicht verschlechtert werden, und der ein gutes Abrasionsverhalten bei gleichzeitiger guter Polierbarkeit auf Hochglanz besitzt.

Gegenstand der Erfindung ist ein Dentalwerkstoff gemäß den Patentansprüchen 1 bis 8 sowie dessen Verwendung gemäß den Ansprüchen 9 bis 11.

Es wurde überraschenderweise gefunden, daß der Einsatz von Fluoriden der Seltenen Erdmetalle (SE-Fluoride) in Dentalwerkstoffen zu den vorstehend erwähnten vorteilhaften Eigenschaften führt. Es sind damit die Trifluoride der Elemente 57 bis 71 gemeint. Obwohl diese Fluoride als Verbindung an sich bekannt sind, war es überraschend, daß sie gegenüber den Oxiden der Seltenen Erdmetalle eine wesentlich verbesserte Transparenz sowie ein gutes Löslichkeitsverfhalten im Mund aufweisen. In der Literatur findet sich dazu kein Hinweis.

Insbesondere haben sich die Fluoride der Seltenen Erdmetalle mit den Ordnungszahlen 59 bis 71 als brauchbar erwiesen, wobei bevorzugte Verbindungen diejenigen der Elemente 66 bis 71 sind. Vorzugsweise wird Ytterbiumfluorid verwendet.

Die Fluoride der Seltenen Erdmetalle werden im allgemeinen als Pulver in den Dentalwerkstoff eingearbeitet. Die mittlere Korngröße der Primärteilchen kann dabei schwanken. Bei einem mikrogefüllten Zahnfüllungsmaterial liegt sie im Bereich von 5 bis 700, insbesondere 20 bis 500, vorzugsweise 50 bis 300 nm. Gegebenenfalls kann die mittlere Primärteilchengröße auch im Bereich von 700 nm bis 5 µm liegen.

Der Gehalt an SE-Fluoriden, bezogen auf das Gesamtgewicht, beträgt zwischen 1 und 50%, insbesondere 5 bis 40%; vorzugsweise liegt er zwischen 10 und 25%. Er hängt insbesondere von der gewünschten Röntgenopazität bzw. Transparenz ab. Es können auch Mischungen der SE-Fluoride verwendet werden.

Im Dentalwerkstoff sind üblicherweise noch andere, nicht röntgenopake, anorganische Bestandteile vorhanden. Als Füllstoffe eignen sich z.B. amorphe Kieselsäuren, insbesondere die pyrogene und gefällte Kieselsäure mit einer BET-Oberfläche von 20 bis 400 m²/g. Insbesondere werden pyrogene Kieselsäuren mit einer BET-Oberfläche von 30 bis 300 m²/g und einer mittleren Korngröße der Primärteilchen von 5 bis 50 nm verwendet, wobei speziell bevorzugte Materialien im bereich zwischen 12 und 50 nm liegen. Kieselsäuren mit einer mittleren Primärteilchengröße von 50 bis 1000, vorzugsweise 100 bis 300 nm können jedoch ebenfalls Verwendung finden.

Die Menge der nicht röntgenopaken Füllstoffe im Dentalwerkstoff ist abhängig von der Menge der verwendeten SE-Fluoride und bewegt sich im allgemeinen im Bereich von 5 bis 84%, insbesondere 10 bis 70%, vorzugsweise 20 bis 50%. Insgesamt beträgt der Gehalt an Füllstoffen (SE-Fluoriden und weiteren anorganischen Verbindungen) 6 bis 85, vorzugsweise 15 bis 85 und insbesondere 30 bis 85 Gew.%.

Die SE-Fluoride und insbesondere die zusätzlichen anorganischen Bestandteile des Dentalwerkstoffes können in üblicher Weise silanisiert sein, um den Verbund zwischen organischer Matrix und anorganischem Füllstoff zu verbessern. Als Haftvermittler eignet sich z.B. γ-Methacryloxypropyl-trimethoxysilan. Die Menge des eingesetzten Haftvermittlers richtet sich nach der Art des Füllstoffes und der gewünschten Viskosität des Dentalwerkstoffes.

Der Dentalwerkstoff muß ferner eine polymerisierbare Vinylverbindung enthalten. Insbesondere eignen sich hierfür monofunktionelle oder polyfunktionelle Methacrylate, die allein oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, aber auch Bis-GMA (Bisphenol-A-glycidylmethacrylat) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri(6-isocyanatohexyl)biuret mit 3 Mol Hydroxyethylmethacrylat und von 1 Mol Trimethylhexamethylendiisocyanat mit 2 Mol Hydroxyethylmethacrylat, die im folgenden als Urethandimethacrylat bezeichnet werden. Der Anteil dieser meist langkettigen Verbindungen im Dentalwerkstoff bewegt sich zwischen 10 und 50 Gew. % Im Prinzip kommen alle für einen Dentalwerkstoff brauchbaren Bindemittel in Frage.

Der Dentalwerkstoff kann je nach Art des verwendeten Katalyators heiß, kalt oder durch Photopolymerisation aushärtbar sein.

Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis-(isobutyroethylester), Benzpinakol und 2,2'-Dimethylbenzpinakol sind geeignet.

Als Katalysatoren für die Photopolymerisation können z.B. Benzophenon und seine Derivate sowie Benzoin und seine Derivate verwendet werden. Beispiele für bevorzugte Photosensibilisatoren sind die α-Diketone wie 9,10-Phenanthrenchinon, Diacetyl, Furil, Anisil, 4,4'-Dichlorbenzil und 4,4'-Dialkoxybenzil. Campherchinon wird besonders bevorzugt verwendet. Die Verwendung der Photosensibilsatoren zusammen mit einem Reduktionsmittel wird bevorzugt. Beispiele für Reduktionsmittel sind Amine wie Cyanethylmethylanilin, Dimethylaminoethylmethacrylat, n-Butylamin, Triethylamin, Triethanolamin, N,N-Dimethylanilin, N-Methyldiphenylamin und N,N-Dimethyl-sym.-xylidin.

Als Katalysatoren für die Kaltpolymerisation werden Radikale liefernde Systeme, z.B. Benozyl- bzw. Lauroylperoxid zusammen mit Aminen wie N,N-Dimethyl-sym.-xylidin oder N,N-Dimethyl-p-toluidin verwendet.

Die Menge dieser Katalysatoren im Dentalwerkstoff liegt üblicherweise zwischen 0,1 bis 5 Gew.%.

Dem Dentalwerkstoff können ferner feinteilige Splitter- oder Perlpolymerisate einverleibt werden, die Homo- oder Copolymere der schon beschriebenen Vinylverbindungen sein können. Diese Homo- bzw. Copolymeren können ihrerseits mit den beschriebenen anorganischen Füllstoffen, auch den röntgenopaken, gefüllt sein. Es wird dazu auf die EP—PS 11 190 und die DE—PS 24 03 211 verwiesen. Ferner kann der Dentalwerkstoff die üblichen Pigmentierungsmittel und Stabilisatoren enthalten.

Vorzugsweise dient der erfindungsgemäße Dentalwerkstoff als Zahnfüllungsmaterial. Dabei soll der Zahnarzt beim Anfertigen von Röntgenbildern in die Lage versetzt werden, aufgrund der Röntgenopazität der Füllung festzustellen, ob sich Sekundärkaries gebildet hat oder nicht. Ein lichthärtendes, röntgenopakes Füllungsmaterial enthält z.B. als Bindemittel ein Urethandimethacrylat bzw. Bis-GMA, Triethylenglycoldimethacrylat als verdünnendes Monomer, SE-Fluorid, z.B. Ytterbiumtrifluorid, pyrogene Kieselsäure mit einer mittleren Größe der Primärteilchen von 40 nm und einer BET-Oberfläche von 50 m²/g, Campherchinon und N,N-Dimethyl-sym.-xylidin als Katalysator sowie Stabilisatoren und Farbpigmente.

Um den Füllungsgrad solcher Füllungsmaterialien zu erhöhen, ist es üblich, z.B. aus Bis-GMA, Triethylenglycoldimethacrylat, Ytterbiumfluorid und der pyrogenen Kieselsäure ein Copolymer herzustellen, dieses als Splitterpolymerisat zu vermahlen und dann in das Füllungsmaterial einzuarbeiten. Die Polymerisation nach dem Legen der Füllung geschieht mit einer handelsüblichen Halogenlampe.

Zahnfüllungsmaterialien werden auch als Zweikomponentenmaterialien hergestellt, die nach dem Anmischen kalt aushärten. Die Zusammensetzung st ähnlich wie bei den lichthärtenden Materialien, nur wird anstatt der Photokatalysatoren in die eine Paste z.B. Benzoylperoxid und in die andere Paste z.B. N,N-Dimethyl-p-toluidin eingemischt. Durch Vermischen etwa gleicher Teile der beiden Pasten erhält man ein Zahnfüllungsmaterial, welches in wenigen Minuten aushärtet.

Wen man bei den letztgenannten Materialien das Amin wegläßt und als Katalysator z.B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird von der Kavität im Munde ein Abdruck genommen und ein Gipsmodell hergestellt. In die Kavität des Gipsmodells wird die Paste eingebracht und das Ganze wird in einem Drucktopf unter Hitze polymerisiert. Das Inlay wird

3

entnommen, bearbeitet und dann im Munde des Patienten in die Kavität einzementiert.

Die Erfindung bezieht sich nicht nur auf den röntgenopaken Dentalwerkstoff, sondern auch auf daraus hergestellte Fertigteile, z.B. künstliche Zähne, Schalen, Inlays etc. Sie wird anhand der nachfolgenden Beispiele erläutert.

Beispiel 1

Es wurden Pasten folgender Zusammensetzung hergestellt.

X % G SE-Fluorid
70-X % G $SiO_2$ (Aerosil OX 50 der Degussa AG) — silanisiert
24% G Urethandimethacrylat
5% G Dekamethylendimethacrylat
1% G Benzoylperoxid

Es wurden Prüfkörper von 1 mm × 10 mm × 12 mm für die Transparenzmessung bzw. von 2 mm × 2 mm × 25 mm für die Messung der Röntgenopazität durch Polymerisation unter Druck bei 120°C hergestellt.

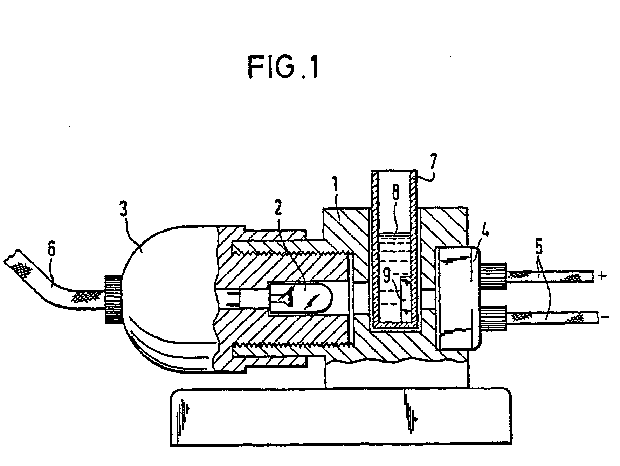

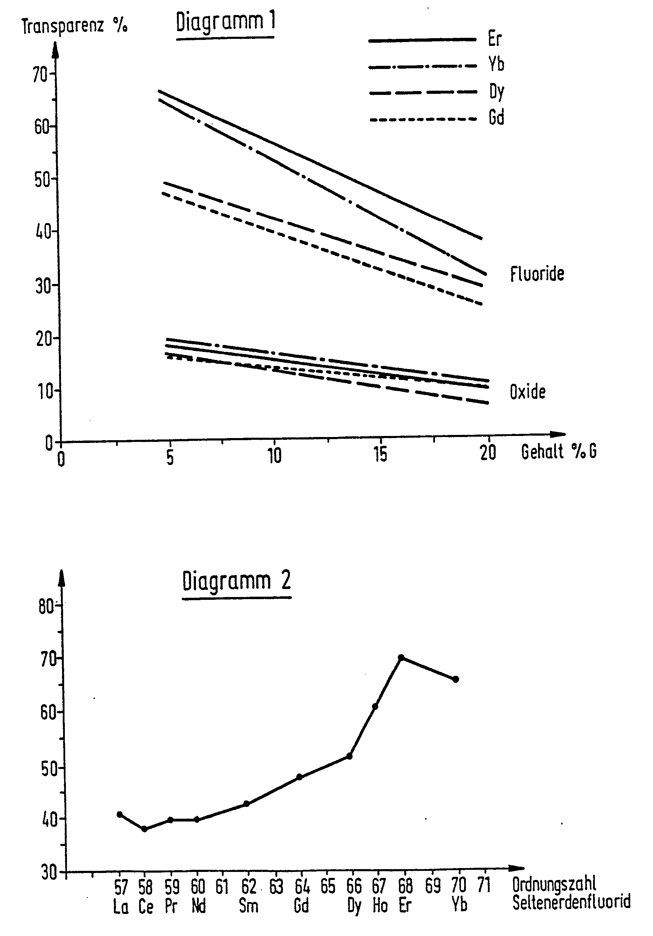

Die Transparenzmessung erfolgte mit der in Abb. 1 gezeigten Transparenz-Prüfeinrichtung. Der Prüfkörper 9 befindet sich in einer mit Wasser 8 gefüllten Glasküvette 7, wobei dieser an der Küvettenwand anliegt, die gegen die Fotozelle 4 abschließt. Die Küvette ist von dem Sockel 1 umgeben. Die Fotozelle 4 ist über die Anschlüsse 5 mit einem Meßgerät verbunden. Die Null-Eichung des Meßgerätes erfolgt automatisch ohne Licht (Lichtquelle 2 im Lampengehäuse 3 ausgeschaltet), während die 100% Eichung ohne Prüfkörper mit Licht (eingeschaltete Lichtquelle 2) erfolgt. Die Lichtquelle wird durch einen 4-Volt-Trafo mit eingebautem Spannungsstabilisator über das Kabel 6 gespeist.

Die Messung der Röntgenopazität wurde folgendermaßen durchgeführt:

Es wurde ein Aluminiumstufenkeil (Reinheit >99,5%) mit einer Länge von 25 mm und einer Breite von 15 mm hergestellt, wobei Abstufungen von 1, 2, 3, 4 und 5 mm vorhanden waren.

Ein Röntgenfilm (ISO 3665, Kodak, Ektaspeed/Phil-X-Film) wurde auf eine Bleiplatte (Dicke >2,0 mm) gelegt. Der Prüfkörper und der Aluminiumstufenkeil wurden nebeinander im Zentrum des Filmes plaziert. Anschließend wurde das Ganze mit einer Dentalröntgenlampe (70 kV Transdent D502 mit Tubus 6 cm φ) bei 70 kVp aus einem Abstand von 100 mm 0,3 Sekunden lang bei 12 mA bestrahlt. Nach dem Entwickeln des Films wurde die durch den Prüfkörper hervorgerufene Schwärzung visuell mit derjeniger des Aluminiumprüfkörpers verglichen. Die Röntgenopazität RO in % Al errechnet sich:

$$RO = \frac{I_{Al}}{I_p} \cdot 100 \ (\% \ Al)$$

$I_p$ = Prüfkörperdicke

$I_{Al}$ = Dicke des Aluminiumkeils mit gleicher Schwärzung wie Prüfkörper

In der Tabelle I sind die Werte aufgetragen. Es zeigte sich, daß die Transparenz mit fallendem Prozentgehalt an SE-Fluorid zunimmt, während das umgekehrte für die Röntgenopazität gilt.

Tabelle II zeigt die Transparenz und Röntgenopazität in Abhängigkeit vom SE-Oxid-Gehalt.

TABELLE I

| SE-F | % G in Paste | Transparenz in % | Röntgenopazität in % Al (visuell) |
|---|---|---|---|
| $LaF_3$ | 5 | 41 | 50 |
| | 10 | -- | -- |
| | 15 | -- | -- |
| | 20 | 23 | 175 |
| $CeF_3$ | 5 | 38 | 75 |
| | 10 | -- | -- |
| | 15 | -- | -- |
| | 20 | 17 | 200 |
| $SmF_3$ | 5 | -- | 70 |
| | 10 | -- | -- |
| | 15 | -- | -- |
| | 20 | 29 | 175 |
| $GdF_3$ | 5 | 48 | 70 |
| | 10 | 39 | 100 |
| | 15 | 33 | 150 |
| | 20 | -- | -- |
| $DyF_3$ | 5 | 52 | 75 |
| | 10 | 40 | 100 |
| | 15 | 35 | 150 |
| | 20 | 30 | 200 |
| $ErF_3$ | 5 | 70 | 75 |
| | 10 | 54 | 125 |
| | 15 | 46 | 150 |
| | 20 | 38 | 200 |
| $YbF_3$ | 5 | 66 | 70 |
| | 10 | 53 | 125 |
| | 15 | 43 | 175 |
| | 20 | 31 | 225 |

-- Wert nicht gemessen

5

TABELLE II

Transparenz und Röntgenopazität in Abhängigkeit vom SE-Oxid-Gehalt

| SE-Oxid | % G in Paste | Transparenz in % | Röntgenopazität in % Al |
|---|---|---|---|
| $Gd_2O_3$ | 5 | 16 | |
| | 7,5 | 15 | |
| | 10 | 13 | |
| | 15 | 11 | |
| | 20 | 9 | |
| $Dy_2O_3$ | 5 | 18 | |
| | 7,5 | 18 | |
| | 10 | 11 | |
| | 15 | 8 | |
| | 20 | 8 | |
| $Er_2O_3$ | 5 | 21 | |
| | 7,5 | 16 | |
| | 10 | 13 | |
| | 15 | 11 | |
| | 20 | 11 | |
| $Yb_2O_3$ | 5 | 21 | 75 |
| | 7,5 | 17 | 100 |
| | 10 | 14 | 150 |
| | 15 | 13 | 200 |
| | 20 | 11 | 225 |

Das Diagramm 1 zeigt die Transparenz in Abhängigkeit vom SE-Oxid- und Fluorid-Gehalt.

(X % $SE_2O_3$ oder $SEF_3$, 70-X % Aerosil (OX 50) silanisiert,
24% Urethandimethacrylat,
5% Dekamethylendimethacrylat,
1% Benzoylperoxid (50%-ig in Phthalat).

Das Diagramm 2 zeigt die Transparenz von SE-Fluoriden nach ihrer Ordnungszahl im Dentalwerkstoff des Beispiels 1.

Beispiel 2
Es wurde ein erfindungsgemäßer Dentalwerkstoff folgender Zusammensetzung hergestellt:

20% G Ytterbiumfluorid
50% G silanisiertes, amorphes $SiO_2$ (BET-Oberfläche 50 m²/g; Aerosil OX 50 der Degussa AG)
24% G Urethandimethacrylat
5,3% G Triethylenglycoldimethacrylat
0,1% G Campherchinon
0,2% G N,N-Dimethyl-sym.-xylidin
0,4% G Farbpigmente und Stabilisatoren.

Die Komponenten wurden in einem Kneter homogen vermischt, wobei eine leichte Erwärmung dies erleichterte. Eine geeignete Beschichtung der Metallteile des Kneters verhinderte dabei einen Metallabrieb.
Die erhaltene Paste ist in hervorragender Weise als Zahnfüllungsmaterial geeignet. Sie härtet bei Bestrahlung mit Licht in kürzester Zeit aus. Sie kann z.B. in schwarzen Spritzen konfektioniert und gelagert werden. Die Röntgenopazität ist hervorragend, ebenso die zahnähnliche Transparenz, die Hochglanzpolierbarkeit sowie das Löslichkeitsverhalten im Mund. Unter letzterer wird eine niedrige Löslichkeit der Füllstoffe bzw. der Füllung im Speichel verstanden.

Beispiel 3

Ein Zweikomponentenzahnfüllungsmaterial wurde wir folgt hergestellt:

| | Peroxidpaste | Aminpaste |
|---|---|---|
| 1. Bis-GMA | 20,4% | 20,4% |
| 2. Triethylenglycoldimethacrylat | 10,6% | 10,6% |
| 3. Ytterbiumfluorid | 15% | 15% |
| 4. Silanisiertes amorphes $SiO_2$ (BET 50 $m^2$/g) | 20% | 20% |
| 5. Vorpolymerisat aus 1—4 | 32,6% | 32,7% |
| 6. Benzoylperoxid | 1,3% | — |
| 7. N,N-Diethanol-p-toluidin | — | 1,2% |
| 8. Farbpigmente und stabilisatoren | 0,1% | 0,1% |

Das Vorpolymerisat No. 5 besteht aus:

20% No. 3

50% No. 4

22% No. 1

8% No. 2

Die Peroxidpaste und die Aminpaste wurden zu gleichen Teilen auf einem Anmischblock ca. 20 Sekunden lang mit einem Spatel gründlich vermischt. Die Paste war als Zahnfüllungsmaterial geeignet und besaß gute Röntgenopazität, Transparenz sowie Hochglanzpolierbarkeit und zeigte ein gutes Löslichkeitsverhalten im Mund.

Beispiel 4

Ein erfindungsgemäßer Dentalwerkstoff wurde wie folgt hergestellt:

20% Dysprosiumtrifluorid
30% Silanisierte amorphe Kieselsäure (BET 130 $m^2$/g; Aerosil 130 der Degussa AG)
17% Bis-GMA
15% Triethylenglycoldimethacrylat
17% Urethandimethacrylat
0,5% Benzoylperoxid
0,5% Farbpigmente und Stabilisatoren.

Die Komponenten wurden in einem Kneter homogen zu einer Paste vermischt. Diese Paste konnte zur Herstellung von Kunststoffinlays verwendet werden, ebenso können aus ihr künstliche Zähne hergestellt werden.

Auch hier waren die Röntgenopazität, die Transparenz, das Löslichkeitsverhalten im Mund sowie die sonstigen physikalischen Eigenschaftern ausgezeichnet.

**Patentansprüche**

1. Röntgenopaker Dentalworkstoff auf Basis von polymerisierbaren organischen Bindemitteln und Verbindungen der Seltenen Erdmetalle sowie gegebenenfalls weiteren anorganischen Verbindungen als Füllstoffen, dadurch gekennzeichnet, daß er ein Fluorid der Seltenen Erdmetalle (Elemente 57 bis 71) des Periodensystems der Elemente) oder ein Gemisch dieser Fluoride enthält.

2. Dentalwerkstoff nach Anspruch 1, dadurch gekennzeichnet, daß er ein Fluorid der Elemente 66 bis 71 des Periodensystems enthält.

3. Dentalwerkstoff nach Anspruch 1 oder 2, dadurch gekennzeichnet, daß er Ytterbiumtrifluorid enthält.

4. Dentalwerkstoff nach einem der Ansprüche 1 bis 3, dadurch gekennzeichnet, daß er ein Fluoride der Seltenen Erdmetalle mit einer mittleren Primärteilchengröße von 5 bis 700 nm enthält.

5. Dentalwerkstoff nach einem der Ansprüche 1 bis 4, dadurch gekennzeichnet, daß er bezogen auf das Gesamtgewicht die Fluoride der Seltenen Erdmetalle in einer Menge von 1 bis 50 Gew.- enthält.

6. Dentalwerkstoff nach einem der Ansprüche 1 bis 5, dadurch gekennzeichnet, daß er als weiteren anorganischen Füllstoff gefällte oder pyrogene Kieselsäure mit einer BET-Oberfläche von 20 bis 400 m$^2$/g und einer mittleren Primärteilchengröße von 5 bis 50 nm enthält.

7. Dentalwerkstoff nach Anspruch 6, dadurch gekennzeichnet, daß er bezogen auf das Gesamtgewicht die Kieselsäure in einer Menge von 5 bis 84% Gew.% enthält.

8. Dentalwerkstoff nach einem der Ansprüche 1 bis 7, dadurch gekennzeichnet, daß er bezogen auf das Gesamtgewicht die anorganischen Füllstoffe insgesamt in einer Menge von 6 bis 85 Gew.% enthält.

9. Verwendung eines Dentalwerkstoffes nach den Ansprüchen 1 bis 8 als 1- oder 2-Komponenten-Zahnfüllungsmaterial oder Dentalzement.

10. Verwendung eines Dentalwerkstoffes nach den Ansprüchen 1 bis 8 als Kronen- und Brückenmaterial oder Zahnprothesenmaterial.

11. Verwendung eines Dentalwerkstoffes nach den Ansprüchen 1 bis 8 zur Herstellung von künstlichen Zähnen, Inlays, Implantaten und Zahnfertigteilen.

**Revendications**

1. Matériau dentaire opaque aux rayons X à base de liants organiques polymérisables et de composés des Terres Rares, ainsi qu'éventuellement d'autres composés inorganiques comme charges, caractérisé en ce qu'il renferme un fluorure des Terres Rares (éléments 57 à 71 de la Classification Périodique des éléments) ou bien un mélange de ces fluorures.

2. Matériau dentaire selon la revendication 1, caractérisé en ce qu'il renferme un fluorure des éléments 66 à 71 de la Classification Périodique.

3. Matériau dentaire selon la revendication 1 ou 2, caractérisé en ce qu'il renferme du trifluorure d'ytterbium.

4. Matériau dentaire selon l'une des revendications 1 à 3, caractérisé en ce qu'il renferme un florure des Terres Rares ayant une taille moyenne des particules primaires comprise entre 5 et 700 nm.

5. Matériau dentaire selon l'une des revendications 1 à 4, caractérisé en ce qu'il renferme les fluorures des Terres Rares en une proportion comprise entre 1 et 50% en poids du poids total.

6. Matériau dentaire selon l'une des revendications 1 à 5, caractérisé en ce qu'il renferme, comme autre charge inorganique, de l'acide silicique précipité ou pyrogène ayant une aire BET de 20 à 400 m$^2$/g et une taille moyenne des particules primaires comprise entre 5 et 50 nm.

7. Matériau dentaire selon la revendication 6, caractérisé en ce qu'il renferme l'acide silicique en une proportion de 5 à 84% en poids du poids total.

8. Matériau dentaire selon l'une des revendications 1 à 7, caractérisé en ce qu'il renferme les charges inorganiques globalement en une proportion de 6 à 85% en poids du poids total.

9. Utilisation d'un matériau dentaire selon l'une des revendications 1 à 8 comme matériau de plombage dentaire à 1 ou 2 composants ou comme ciment dentaire.

10. Utilisation d'un matériau dentaire selon l'une des revendications 1 à 8 comme matériau pour couronnes et bridges ou comme matériau pour prothèses dentaires.

11. Utilisation d'un matériau dentaire selon l'une des revendications 1 à 8 pour la fabrication de dents artificielles, d'inlays, d'implants et d'éléments dentaires préfabriqués.

**Claims**

1. Radiopaque dental material based on polymerizable organic binders and compounds of the rare earth metals, and also optionally further inorganic compounds as fillers, characterized in that it contains a fluoride of the rare earth metals (elements 57 to 71 of the periodic system of elements) or a mixture of these fluorides.

2. Dental material according to claim 1, characterized in that it contains a fluoride of elements 66 to 71 of the periodic system.

3. Dental material according to claim 1 or 2, characterized in that it contains ytterbium trifluoride.

4. Dental material according to one of claims 1 to 3, characterized in that it contains a rare earth metal fluoride with an average primary particle size of 5 to 700 nm.

5. Dental material according to one of claims 1 to 4, characterized in that it contains rare earth metal fluorides in a quantity of 1 to 50% by weight, referred to the total weight.

6. Dental material according to one of claims 1 to 5, characterized in that as a further inorganic filler, it contains precipitated or pyrogenic silicic acid with a BET surface of 20 to 400 m$^2$/g and an average primary particle size of 5 to 50 nm.

7. Dental material according to claim 6, characterized in that it contains the silicic acid in a quantity of 5 to 84% by weight, referred to the total weight.

8. Dental material according to one of claims 1 to 7, characterized in that it contains the inorganic fillers in total in a quantity of 6 to 85% by weight, referred to the total weight.

9. Use of a dental material according to claims 1 to 8 as a one or two-component tooth-filling material or dental cement.

10. Use of a dental material according to claims 1 to 8 as a crown or bridge material or a dental prosthesis material.

11. Use of a dental material according to claims 1 to 8, for the production of dentures, inlays, implants and finished dental parts.

FIG.1

Diagramm 1

Diagramm 2